# EUROPEAN PATENT SPECIFICATION

(11) **EP 1 131 348 B1**
(45) Date of publication and mention of the grant of the patent: **07.03.2007**
(21) Application number: 00962685.4
(22) Date of filing: 15.09.2000
(51) Int. Cl.: C07K 14/47, A61P 35/00, A61P 31/18, A61K 38/17

(54) **REGULATORY/UNFOLDING PEPTIDES OF EZRIN**
REGULATORISCHE/ENTFALTENDE PEPTIDE VON EZRIN
PEPTIDES D'EZRINE DE REGULATION/A DEPLOIEMENT

(30) Priority: 17.09.1999 GB 9921881
(43) Date of publication of application: 12.09.2001
(60) Divisional application: 06009089.1
(73) Proprietor: Holms, Rupert Donald, London NW1 7SX (GB)
(72) Inventor: Holms, Rupert Donald, London NW1 7SX (GB)
(74) Representative: Perry, Robert Edward
(86) International application number: PCT/GB2000/003566
(87) International publication number: WO 2001/025275

(56) References cited:
- WO-A-95/33768
- WO-A-97/12975
- WO-A-99/47150
- GOULD, KATHLEEN L. ET AL.: "CDNA CLONING AND SEQUENCING OF THE PROTEIN-TYROSINE KINASE SUBSTRATE EZRIN REVEALS HOMOLOGY TO BAND 4 1" EMBO J (1989) 8(13) 4133-42, XP002162130

## Description

### Field of the Invention

This invention relates to peptides having utility in the treatment of infections, diseases, cancer and conditions of the immune system.

### Background of the Invention

Ezrin is a member of the ERM (ezrin-radixin-moesin) family of proteins which play structural and regulatory roles in a wide range of cell types. There is considerable evidence to indicate that ezrin regulates the structure of the cortical cytoskeleton to control cell surface topography.

Detailed analysis of the secondary structure of ezrin shows that there are three main structural domains: a N-terminal domain from amino acids 1 to 300, a highly charged alpha domain from amino acids 300 to 470 and C terminal domain from amino acids 470 to 585.

WO95/33768 discloses that a 14 amino-acid peptide identified as HEP1, i.e. TEKKRRETEREKE (identical to amino acids 324-337 of human ezrin) which has a 70% identity to the C terminus of gp120, could inhibit HIV replication in vivo in man. At the time I believed that the observed anti-HIV effect was due to the induction of immunological tolerance to autoreactive immune responses induced by the C terminus of HIV gp120.

Gould et al, EMBO J. (1989) 8(13): 4133-32, discloses peptides identical to parts of the Hepreceptor.

### Summary of the Invention

The soluble conformation of ezrin found in the cytoplasm comprises two adjacent alpha helical domains which are folded together at a hinge region (M339-M340) into two anti-parallel helices stabilised by complementary side-chain charges of the primary amino acid sequence. This invention is based on the understanding that the positively and negatively charged side-chains of the amino acid sequence of Hepreceptor Domain A are complementary to the positively and negatively charged side-chains of the amino acid sequence of the Hepreceptor Domain B.

In the activated open conformation of ezrin, the interaction of Domain A and Domain B of the Hepreceptors of two different ezrin molecules allows the formation of anti-parallel dimers. In addition to the antiparallel dimers of ezrin which form below the cell surface, I have determined that these dimers can form between a Hepreceptor exposed on the surface on one cell with a Hepreceptor exposed on the surface of another cell. When the two Hepreceptors make contact during close association of two cell surfaces, an activation signal is initiated in both cells. Any charged molecule that partially mimics the interaction between the side-chains charges of Domain A and Domain B of the Hepreceptor will give rise to a medically useful biological response.

The invention is based also on a realisation that the anti-HIV activity of HEP1 is due to its binding to Hepreceptor Domain B and the induction of a novel immune response.

According to the present invention, a peptide for the treatment of infectious diseases, cancer or a condition of the immune system, comprises at least 5 consecutive amino acids of the following 34 amino acid sequence:
M R E K E E L M L R L Q D Y(p) E E K T K K A E R E L S E Q I Q R A L Q.

This 34 amino acid sequence is the Hepreceptor-Domain B (amino acid numbers 340-373 of human ezrin), in which Tyrosine 353 [Y] may be phosphorylated to phosphotyrosine [Yp] in the membrane-associated conformation of ezrin. I have determined that Domain B of the Hepreceptor is the site on ezrin to which HIV gp120 binds during infection of the brain (HIV gp120 binds to Hepreceptor Domain B using its charged C terminal amino acids which have a 70% identity to part of Hepreceptor Domain A). Novel charged molecules which bind to the Hepreceptor may be useful in treating HIV-related dementia.

According to a further aspect of the invention, a peptide of the invention is used for the manufacture of a medicament for the treatment of infectious disease, cancer or a condition of the immune system.

### Description of the Invention

Peptides of the invention preferably have 5 to 13 amino acids. Exemplary peptides of the invention are the following Hepreceptor Domain A-binding peptides:

| | |
|---|---|
| Rupe2024: | KEELM |
| Rupe2032: | KEELMLRLQDYEE |
| Rupe2032p: | KEELMLRLQDYpEE |
| Rupe2132: | EELMLRLQDYEE |
| Rupe2132p: | EELMLRLQDYpEE |
| Rupe2232: | ELMLRLQDYEE |
| Rupe2232p: | ELMLRLQDYpEE |
| Rupe2428: | MLRLQ |
| Rupe2832: | QDYEE |
| Rupe2832p: | QDYpEE |

Other peptides or other charged molecules which bind to Domain A of the Hepreceptor are likely to be biologically active. These peptides or other charged molecules can be administered orally and by other routes, for the treatment of various infectious diseases and cancer.

### HOW TO MAKE

Peptides used in this invention may be synthesised for example, using a solid phase method using either Boc or Fmoc chemistry or any other practical route for peptide synthesis known to those skilled in the art of peptide synthesis.

Stepwise solid phase synthesis with Boc-amino acids can be performed based on the method of Merrifield; (Journal of American Chemical Society 85 2149-2154). The following compounds can be used (Novabiochem resin: Boc-Glu(OBzl)-PAM, Amino acids: Boc-Lys (2Cl-Z-)OH, Boc-Glu(OBzl)OH, Boc-Arg(Tos)OH, Boc-Val-OH, Boc-Thr (Bzl)-OH, Solvents: DMF (Rathburn), Dichloromethane (BDH), Ethylacetate (BDH), Reagents: HBTU (Phase Separations Ltd), p-Cresol (Lanchester) TFA (Halocarbon Products Corporation) HF (BOC) DIEA (Fluka). Recommended reactive side chain protecting groups for Boc-amino acids are:, Arg (Tos), Asn (Xan), Asp (OcHxl), Glu (OcHxl) Gln (Xan) or Gln, His (DNP), Lys (CIZ), Serine (Bzl) Tyr (BrZ) Trp (CHO). The abbreviations have the following meanings: DCC= Dicyclohexyldarbodilmide, DIC=Diisopropylcarbodiimide, DCM=Dichloromethane, DMF=Dimethylformamide, TFA=Trifluoracetic acid, Boc= t-Butyloxycarbonyl, HOBT=Hydroxybenzotriazole, DIEA=Diisopropylethylamine, DCU=Dicyclohexylurea.

For example, boc synthesis of a peptide of this invention could be performed as follows: HBTU activation/in situ neutralization on 0.5 mmol scale uses 0.5 mmol of resin and a three fold excess of activated Boc amino acid. Boc amino acid and activating reagent (HBTU) should be used in equimolar quantities ie 2mmol each in this case equals a 3x excess. DIEA is used to both neutralise the resin for coupling and to activate the Boc-amino acid. (Hence 2.5 mmol is used, 1 equivalent Boc-aa and 1 equivalent resin). Reagents: 0.5M HBTU in DMF (MW=379, 0.5M=18.95g in 100ml, note it is not light stable) requires 2mmol=4ml and 2.5 mmol DIEA=0.46ml (MW=129, d=0.742). Activation of aminoacids: Boc-amino acid should be activated only immediately prior to addition to the resin, especially in the case of Arg (Tos). For all Boc amino acids: weigh 2 mmol Boc amino acid into a 20 ml glass sample bottle. Add 4ml 0.5M HBTU solution and shake to dissolve solid. Add 0.46 ml DIEA and mix (some colour change may be observed). Method: wash resin with DMF, remove Boc-protecting group with 100% TFA- Shake twice for 1 minute draining in between, drain, flow wash with DMF for 1 minute, drain, add activated amino acid solution, shake for 10 minutes, then take sample and perform the ninhydrin test to determin coupling efficiency. On completion of the synthesis flow wash with DMF, then DCM and dry. The synthesis of the first and every subsequent level of peptide construction is achieved using a three fold excess of HBTU activated Boc-amino acids in DMF. In all couplings, the coupling efficiency should be more than 99% as indicated by quantative ninhydrin testing. Deprotection of the N-termini is performed in 100% TFA. The resin peptide is carefully flow-washed before and after the deprotection. After the last coupling and removal of the Boc-protection, the peptide resin is washed with dichloromethane and dried by air. The peptide is removed from the resin support by the high HF method (2 g resin peptide, 2g cresol, 20ml HF, 1.5 h at -5°C) to yield the crude peptide which is precipitated with ethylacetate (100ml) and redissolved in 6M guanidine HCL-0.1M TRIS solution (20ml).

The peptide can be purified as follows using an analytical HPLC separation on a Vydac C₁₈ 5 RAC column. HPLC grade acetonitrile (aldrich) and water is filtered through a membrane filter and degassed with helium flow prior to use. Analytical separation achieved with a solvent gradient beginning with 0% acetonitrile, increasing constantly to 60% acetonitrile at 20 minutes, staying at this concentration for twenty minutes and decreasing steadily to 0% acetonitrile for 10 minutes at a constant flow of 1.2 ml per minute. Preparative separation of peptide achieved on a TSK-GEL preparative C₁₈ column. Separation is achieved with a solvent gradient beginning with 0% acetonitrile, increasing constantly to 18% acetonitrile at 60 minutes, then 60% acetonitrile for 80 minutes, staying at this concentration for 30 minutes at a constant flow of 8 ml per minute. The gradient can be controlled by two microprocessor controlled Gilson 302 single piston pumps. Compounds can be detected with a Waters 486 Tunable Absorbance Detector at 214 nm and analytical chromatographs recorded with an HP laserjet 5L. A Holochrome UV-VIS detector 220 nm for preparative chromatographs can be recorded with an LKB 2210 single channel recorder. Capillary Electorphoresis quality control can be carried out using Waters Quanta 4000 equipment using a phosphate buffer (75 microM) pH2.5 run at 15 kV, sample time 20 seconds, loaded by hydostatic injection on 60 cm column, run time 12 minutes. The yield for 1 g 0.46 mmol resin synthesis should be about 250 mg pure peptide

Alternative solution synthesis methods may also be used to produce larger quantities of the peptides of this invention. Protected trimer fragments can be obtained using stepwise synthesis by the active esters method known to those skilled in the art of peptide synthesis. The fragments are then assembled using DCC/HOBT after removal of relevant C and N terminal protective groups. After removal of all protective groups the crude peptide is partially purified on SP-Sephadex-C25 ion exchange chromatography followed by preparative HPLC then lyophilised.

### HOW TO USE

0.01 to 1000 mg of lyophilised peptide may be dissolved in 1-10 ml distilled water and administered orally or vaginally. 0.01 to 1000 mg may be formulated in to a pill or capsule or suppository with carriers used commonly by those skilled in the art of pill or capsule or suppository manufacture and administered orally or vaginally or anally. A filter sterilized solution of between 0.001 and 100 milligrams of peptide in distilled water may be injected subcutaneously or intravenously or intramuscularly.

The following Examples serve to illustrate the invention only, and should not be construed as limiting it in any way.

### Example 1A

I have demonstrated that Hepreceptor peptides have significant adjuvant activity and this is demonstrated by enhancing the IgG antibody response to Ovalbumin in mice, and by enhancing the antibody-dependent cytotoxic response in Thymus to Sheep Red Blood Cells (SRBC) in mice, using Rupe2032.

The IgG response in mice two weeks after an injection of 50 micrograms of Ovalbumin plus various concentrations of Rupe 2032 determined. IgG response was measured by Optical Density (OD) of a 1 to 100 dilution of mouse sera in the presence of Ovalbumin. The following table reports IgG response recorded as OD:

| **Peptide code** | **Control** | | **IP injection (µg/mouse)** | | |
|---|---|---|---|---|---|
| | **0** | **0.01** | **0.1** | **1** | **10** |
| Rupe2032 | 0.127 | 0.509 | 0.606 | 0.327 | 0.203 |

The next table reports IgG response recorded as OD then data rebased relative to 100 for each control:

| **Peptide code** | **Control** | | **IP injection (µg/mouse)** | | |
|---|---|---|---|---|---|
| | **0** | **0.01** | **0.1** | **1** | **10** |
| Rupe2032 | 100 | 401 | 477 | 257 | 160 |

### Example 1B

The influence of Hepreceptor peptides on the activation of antibody-forming cells against sheep erythocytes (SRBC) in Mice (CBA - C57Bl Fi hybrids, 2months old, weight 18-22 grams) was determined. Mice were first injected intraperitoneally using either 0.5 ml sterile saline as a control or Rupe 2032 in the same volume of saline. 30 minutes after the injection of peptides, a cell suspension containing 5 million SRBC was administered intraperitoneally to each mouse. Four days after the immunisation, the mice were killed by cervical dislocation and the spleens were obtained aseptically. Each spleen was homogenised in 2 ml of Medium 199, then 100 ml of this suspension was put into 1 ml of prepared agarose in Medium 199 (stored in water bath at 48°C), and SRBC suspension was added, resulting in a final concentration of 1% agarose. The 1 ml of agarose-cell mixture was agitated then transferred to petri dish to set. When the agarose became solid, the petri dish was incubated for 1 hour at 37°C then 0.5 ml of 1-in-20 diluted guinea pig serum in Medium 199 was added on top of agarose gel as a source of complement. The incubation was continued for another 1 hour. The dishes were then visualised using an 8x binocular microscope and the number of plaques counted (each plaque equivalent to one antibody-secreting mouse spleen cell). The results were expressed both as the number of antibody-secreting cells per one million nucleated spleen cells and the number of antibody-secreting cells per whole spleen. As no mitogenic effect was observed (Hepreceptor peptide administration resulted in normal sized mouse spleens), these two calculations gave similar results.

The following table reports averaged data (from thirty mice per data point) of the number of antibody forming cells per one million nucleated spleen cells. The data was re-based relative to 100 for each control group.

| **Peptide code** | **Control** | | **IP injection (µg/mouse)** |
|---|---|---|---|
| | **0** | **1** | **10** |
| Rupe2032 | 100 | 236 | 232 |

## Claims

1. A peptide for the treatment of infectious disease, cancer or a condition requiring stimulation of the immune response, wherein the peptide comprises at least 5 consecutive amino acids of the following 34 amino acid sequence:
M R E K E E L M L R L Q D Y (p) E E K T K K A E R E L S E Q I Q R A L Q

2. A peptide of claim 1, which has 5 to 13 amino acids.

3. A peptide of claim 1, comprising the amino acid sequence
KEELM

4. A peptide of claim 1, comprising the amino acid sequence
KEELMLRLQDYEE

5. A peptide of claim 1, comprising the amino acid sequence
KEELMLRLQDYpEE

6. A peptide of claim 1, comprising the amino acid sequence
EELMLRLQDYEE

7. A peptide of claim 1, comprising the amino acid sequence
EELMLRLQDYpEE

8. A peptide of claim 1, comprising the amino acid sequence
ELMLRLQDYEE

9. A peptide of claim 1, comprising the amino acid sequence
ELMLRLQDYpEE

10. A peptide of claim 1, comprising the amino acid sequence
MLRLQ

11. A peptide of claim 1, comprising the amino acid sequence
QDYEE

12. A peptide of claim 1, comprising the amino acid sequence
QDYpEE

13. Use of a peptide of any preceding claim, for the manufacture of a medicament for the treatment of infectious disease, cancer or a condition requiring stimulation of the immune response.

## Patentansprüche

1. Peptid zur Behandlung von infektiöser Erkrankung, Krebs oder einem Zustand, der Stimulation der Immunantwort erfordert, wobei das Peptid mindestens 5 aufeinanderfolgende Aminosäuren der folgenden 34 Aminosäuren-Sequenz umfasst:
M R E K E E L M L R L Q D Y₍ₚ₎ E E K T K K A E R E L S E Q I Q R A L Q

2. Peptid nach Anspruch 1, das 5 bis 13 Aminosäuren aufweist.

3. Peptid nach Anspruch 1, umfassend die Aminosäuresequenz
KEELM

4. Peptid nach Anspruch 1, umfassend die Aminosäuresequenz
KEELMLRLQDYEE

5. Peptid nach Anspruch 1, umfassend die Aminosäuresequenz
KEELMLRLQDYpEE

6. Peptid nach Anspruch 1, umfassend die Aminosäuresequenz
EELMLRLQDYEE

7. Peptid nach Anspruch 1, umfassend die Aminosäuresequenz
EELMLRLQDYpEE

8. Peptid nach Anspruch 1, umfassend die Aminosäuresequenz
ELMLRLQDYEE

9. Peptid nach Anspruch 1, umfassend die Aminosäuresequenz
ELMLRLQDYpEE

10. Peptid nach Anspruch 1, umfassend die Aminosäuresequenz
MLRLQ

11. Peptid nach Anspruch 1, umfassend die Aminosäuresequenz
QDYEE

12. Peptid nach Anspruch 1, umfassend die Aminosäuresequenz
QDYpEE

13. Verwendung eines Peptids nach irgendeinem vorhergehendem Anspruch zur Herstellung eines Arzneimittels zur Behandlung von infektiöser Erkrankung, Krebs oder einem Zustand, der Stimulation der Immunantwort erfordert.

## Revendications

1. Peptide pour le traitement d'une maladie infectieuse, d'un cancer ou d'un état exigeant une stimulation de la réponse immunitaire, dans lequel le peptide comprend au moins 5 acides aminés consécutifs de la séquence suivante de 34 acides aminés
MREKEELMLRLQDY (p) EEKTKKAERELSEQIQRALQ

2. Peptide selon la revendication 1, qui possède 5 à 13 acides aminés.

3. Peptide selon la revendication 1, comprenant la séquence d'acides aminés KEELM.

4. Peptide selon la revendication 1, comprenant la séquence d'acides aminés KEELMLRLQDYEE.

5. Peptide selon la revendication 1, comprenant la séquence d'acides aminés KEELMLRLQDYpEE

6. Peptide selon la revendication 1, comprenant la séquence d'acides aminés EELMLRLQDYEE

7. Peptide selon la revendication 1, comprenant la séquence d'acides aminés EELMLRLQDYpEE

8. Peptide selon la revendication 1, comprenant la séquence d'acides aminés ELMLRLQDYEE

9. Peptide selon la revendication 1, comprenant la séquence d'acides aminés ELMLRLQDYpEE

10. Peptide selon la revendication 1, comprenant la séquence d'acides aminés MLRLQ

11. Peptide selon la revendication 1, comprenant la séquence d'acides aminés QDYEE

12. Peptide selon la revendication 1, comprenant la séquence d'acides aminés QDYpEE

13. Utilisation d'un peptide selon l'une quelconque des revendications précédentes, pour la fabrication d'un médicament pour le traitement d'une maladie infectieuse, d'un cancer ou d'un état exigeant une stimulation de la réponse immunitaire.
